# EUROPÄISCHE PATENTSCHRIFT

(11) **EP 1 143 867 B1**
(45) Veröffentlichungstag und Bekanntmachung des Hinweises auf die Patenterteilung: **31.07.2002**
(21) Anmeldenummer: 99960994.4
(22) Anmeldetag: 24.11.1999
(51) Int. Cl.: A61B 17/58, A61B 17/72

(54) **FIXATIONSSYSTEM FÜR KNOCHEN**
BONE FIXATION SYSTEM
SYSTEME DE FIXATION POUR OS

(30) Priorität: 19.12.1998 DE 19858889
(43) Veröffentlichungstag der Anmeldung: 17.10.2001
(73) Patentinhaber: Wolter, Dietmar, Prof. Dr., D-21033 Hamburg (DE)
(72) Erfinder: Wolter, Dietmar, Prof. Dr., D-21033 Hamburg (DE)
(74) Vertreter: Siemons, Norbert, Dr.-Ing. Patentanwälte Hauck, Graalfs, Wehnert, Döring, Siemons
(86) Internationale Anmeldenummer: EP9909065
(87) Internationale Veröffentlichungsnummer: WO0036984

(56) Entgegenhaltungen:
- WO-A-97/09000
- DE-A- 4 343 117
- DE-A- 19 629 011
- US-A- 5 534 032
- US-A- 5 810 823

## Beschreibung

Die Erfindung betrifft ein Fixationssystem für Knochen mit einem Verbindungsträger und mindestens einer Knochenschraube oder mindestens einem Verriegelungsbolzen.

Derartige Fixationssysteme werden in der Osteosynthese verwendet, wobei die Knochenschrauben mit den Knochenenden verbunden werden und der Verbindungsträger den Bruch überbrückt. Der Verbindungsträger kann insbesondere eine Knochenplatte, ein Marknagel oder ein Fixateur sein. Hierbei ist es wünschenswert, die Knochenschrauben in Anpassung an die Gegebenheiten des zu verbindenden Knochenteiles, zur optimalen Ausrichtung auf die Fragmente oder zum Ausgleich von Zielfehlern unter verschiedenen Winkeln in den Verbindungsträger einbringen zu können.

Hierzu haben bei einem bekannten Fixationssystem die Knochenschrauben Köpfe mit etwa halbkugelförmiger Sitzfläche, denen eine Sitzfläche in Durchgangslöchern einer Knochenplatte zugeordnet ist. Wenn beispielsweise bei einer Unterschenkelfraktur die beiden Knochenstücke miteinander verbunden werden müssen, wird die metallische Knochenplatte auf die eingerichteten Knochenstücke gelegt. Danach werden die Schrauben so in den Knochen eingedreht, daß die Sitzflächen der Schraubenköpfe und der Plattenlöcher in Anlage aneinander kommen und die Platte gegen den Knochen gepreßt wird. Daraus resultiert eine Verbindung von Knochenteilen, Knochenplatte und Knochenschrauben. Jedoch hat sich gezeigt, daß eine Lockerung der Verbindungen von Knochenschrauben und Knochenplatten stattfinden kann. Eine Ursache liegt in der ungenügenden Stabilität der Winkelverbindungen von Knochenschrauben und Knochenplatte, die durch Reibkräfte zwischen Schraubenkopf und Plattenloch gesichert sind.

Eine winkelstabile Verbindung von Knochenschraube und Knochenplatte führt hingegen zu einem Stabilitätsgewinn der gesamten Montage. Es sind verschiedene Lösungen bekannt, eine derartige stabile Verbindung zu erreichen. Gemäß EP-A-10 201 024 kann dies beispielsweise dadurch geschehen, daß der Knochenplatte eine Druckplatte zugeordnet ist, die mit den Schraubenköpfen verspannbar ist und diese in einer gewählten Winkellage fixiert. Solche aufwendigen Fixationssysteme mit einer Druckplatte sind aufgrund ihres verhältnismäßig großen Volumens in der Anwendbarkeit eingeschränkt.

Eine andere Lösung besteht gemäß WO/89 041 50 darin, den Schraubenkopf in einem Schlitzbereich mit einer Spreizschraube aufzuweiten und hierdurch im Plattenloch einzupressen. Dabei haben der Schraubenkopf bzw. ein diesen umgebender Einsatz sowie das Plattenloch kugelförmige Sitzflächen, die eine Ausrichtung unter verschiedenen Winkeln ermöglichen. Dieses Fixationssystem ist ebenfalls in Herstellung und Anwendung aufwendig.

Außerdem ist schon bekannt, den Schraubenkopf mit einem Außengewinde und das Plattenloch mit einem Innengewinde zu versehen. Wird nun die Schraube in den Knochen eingedreht, so kommt es durch die Gewindeverbindung zu einer winkelstabilen Ausrichtung von Platte und Schraube. Diese Lösung hat jedoch den gravierenden Nachteil, daß die Schraube nicht in einem beliebigen Winkel, sondern nur in der durch die Gewindeachsen vorgegebenen Ausrichtung in das Plattenloch eingebracht werden kann.

Dieser Nachteil wird gemäß DE 43 43 117 A1 in der Weise überwunden, daß die Mittel zum Festlegen der Knochenschraube in einem bestimmten Winkel zur Knochenplatte eine durch Eindrehen der Knochenschraube in einem bestimmten Winkel von einem vorgeformten Gewinde an mindestens einer Sitzfläche gebildete Gewindeverbindung der Sitzflächen von Knochenplatte und Knochenschraube aufweisen. Dabei können sowohl die Sitzfläche der Knochenschraube als auch die Sitzfläche der Knochenplatte ein vorgeformtes Gewinde aufweisen. Beim Eindrehen der Knochenschraube kommt es durch das mindestens eine Gewinde zur Umformung des Materials in den Sitzflächen und somit zur Ausbildung einer Gewindeverbindung in der jeweiligen Schraubrichtung.

Diese Lösung hat jedoch insbesondere bei Verwendung von dickeren Platten den erheblichen Nachteil, daß die Kraft zur Umformung des Materials beträchtlich ist und diese intraoperativ nicht immer problemlos aufgebracht werden kann. Um dieses möglich zu machen, wurde daher in der DE 196 29 011 A1 ein zusätzlicher, bolzenförmiger Vorformer zum Ausrichten in Knochenbohrungen mit einem Außengewinde zum Vorformen eines Gewindes in dem Durchgangsloch eines Verbindungsträgers angegeben. Dieses Vorformen eines Gewindes hat jedoch den Nachteil, daß es einen zusätzlichen Arbeitsgang erfordert und zum Anfall von Abrieb- und Spanpartikeln aus dem Wandbereich des Durchgangsloches führt. Untersuchungen haben gezeigt, daß es bei der Verwendung von Reintitan im Durchgangsloch einer Unterschenkelplatte zur Spanbildung von 0,0001 g pro Durchgangsloch kommt. Eine ähnliche Menge ergibt sich auch bei herkömmlichen Metallplatten. Dies ist sowohl aus Gründen der Verträglichkeit als auch aufgrund der Tatsache, daß es sich hier um Fremdkörper handelt, nachteilig zu beurteilen.

Davon ausgehend liegt der Erfindung die Aufgabe zugrunde, ein Fixationssystem für Knochen zu schaffen, bei dem das Eindrehen der Knochenschrauben in verschiedenen Winkelstellungen in den Verbindungsträger erleichtert ist, daß dennoch eine stabile Fixierung der Knochenschraube in ihrer Winkelstellung zum Verbindungsträger erreicht wird, wobei der Abrieb- und Spananfall beim Bilden der Gewindeverbindung erheblich vermindert ist.

Die Aufgabe wird durch Fixationssysteme mit den Merkmalen des Anspruches 1 und ein Fixationssystem mit den Merkmalen des Anspruches 25 gelöst. Vorteilhafte Ausgestaltungen des Fixationssystems sind in den Unteransprüchen angegeben.

Bei dem Fixationssystem gemäß Anspruch 1 weisen die Mittel zum Festlegen der mindestens einen Knochenschraube eine durch Eindrehen der Knochenschraube in einem bestimmten Winkel von einem vorgeformten Gewinde unterhalb mindestens einer der Sitzflächen von Knochenschraube und Verbindungsträger durch Umformung gebildete Gewindeverbindung unterhalb der Sitzflächen von Knochenschraube und Verbindungsträger auf. Dadurch, daß die Gewindeverbindung nicht in den Sitzflächen, sondern unterhalb derselben unter Beteiligung mindestens eines vorgeformten Gewindes durch Umformung gebildet wird, lassen sich die von der Umformung betroffenen Bereiche der Verbindung beträchtlich reduzieren. Infolgedessen wird das Eindrehen der Knochenschraube erleichtert und der Zwischenschritt des Gewindeformens mittels eines speziellen Gewindeformers überflüssig. Außerdem wird die Produktion von Abriebpartikeln und Spanmaterial bis auf ein unwesentliches Ausmaß reduziert. Dennoch wird durch die Sitzflächen und die Gewindeverbindung eine winkelstabile Schraubverbindung mit der erwünschten Festigkeit erreicht. Diese kann durch einen sich infolge der Umformung ergebenden Kraftschluß (Reibschluß) und/oder Stoffschluß (Reibschweißen) in der Gewindeverbindung gesichert sein.

Das vorgeformte Gewinde kann sich unterhalb beider Sitzflächen oder unterhalb nur einer der beiden Sitzflächen befinden, so daß die Gewindeverbindung unter Umformung mindestens eines vorgeformten Gewindes bzw. eines gewindefreien Bereiches unter einer Sitzfläche gebildet wird. Bevorzugt wird die Gewindeverbindung von einem vorgeformten Gewinde unterhalb der einen Sitzfläche und mindestens einem mindestens teilweise umlaufenden, durch Eindrehen des vorgeformten Gewindes leicht umformbaren Vorsprung unterhalb der anderen Sitzfläche gebildet. Dabei kann der Vorsprung eine Ausgestaltung haben, die dessen Umformung mittels des vorgeformten Gewindes erleichtert, beispielsweise grat- oder lippenartig ausgestaltet sein. Auch kann der Vorsprung - im Unterschied zu einem schraubenlinienförmig umlaufenden Gewinde - auch symmetrisch auf einer Kreislinie um die Lochachse angeordnet sein, wodurch der Platzbedarf für den Vorsprung verringert wird und die Abhängigkeit der Eindrehkraft von der Lage der Einschraubachse zur Lochachse verringert wird.

Bevorzugt sind die Sitzflächen sphärisch oder konisch, wodurch das Einschrauben und Plazieren der Knochenschrauben in verschiedenen Winkelstellungen im Verbindungsträger begünstigt wird. Weiterhin bevorzugt ist die eine Sitzfläche an der Unterseite eines Schraubenkopfes der Knochenschraube ausgebildet. Dabei kann die Gewindeverbindung mit einem Abschnitt des Schraubenkopfes unterhalb der einen Sitzfläche gebildet sein, der dafür das vorgeformte Gewinde aufweisen kann. Auf diese Weise werden Sitzfläche und Mittel zum Festlegen platzsparend an der Knochenschraube ausgebildet.

Gemäß einer weiteren bevorzugten Ausgestaltung ist die andere Sitzfläche in dem Durchgangsloch des Verbindungsträgers ausgebildet. Dabei kann die Gewindeverbindung mit einem Abschnitt des Durchgangsloches unterhalb der anderen Sitzfläche gebildet sein. Bevorzugt weist dieser Abschnitt den mindestens einen umformbaren Vorsprung unterhalb der anderen Sitzfläche auf. Damit werden andere Sitzfläche und Mittel zum Festlegen auch im Verbindungsträger platzsparend untergebracht. Mit den vorerwähnten Ausbildungen von Knochenschraube und Verbindungsträger können Knochenplatten, die beispielsweise eine Dicke von einigen Millimetern haben, gebildet werden, ohne daß der Schraubenkopf über die Oberseite der Knochenplatte übersteht.

Gemäß einer weiteren Ausgestaltung ist in einem Abstand von dem umformbaren Vorsprung mindestens ein weiterer, mindestens teilweise umlaufender, durch Eindrehen des vorgeformten Gewindes umformbarer Vorsprung unterhalb der anderen Sitzfläche angeordnet. Dann sitzt die Knochenschraube nach dem Eindrehen in mehreren umformbaren Vorsprüngen fest, wodurch die Winkelverbindung weiter stabilisiert wird. Zwecks weiterer Stabilisierung der Winkelverbindung kann auch oberhalb der anderen Sitzfläche im Durchgangsloch mindestens noch ein weiterer, mindestens teilweise umlaufender, durch Eindrehen der Knochenschraube umformbarer Vorsprung angeordnet sein. Dieser Vorsprung kann mit dem Schraubenkopf zusammenwirken. Hierdurch kann eine - im Querschnitt - schwalbenschwanzförmige Gestaltung der Wand des Durchgangsloches gegeben sein, die eine günstigeren Schraubenkopf- und Lochwandkontakt ergibt, wodurch die Stabilisierung durch die Knochenschraube in ihrer Winkellage weiter stabilisiert wird.

Bei verhältnismäßig dünnen Verbindungsträgern, z.B. Knochenplatten, wie sie vorzugsweise im Bereich des Sprunggelenkes, der Halswirbelsäule oder des körperfernen Unterarmendes eingesetzt werden, die beispielsweise eine Dicke von 1 bis 2 mm haben können, kann der Verbindungsträger um das Durchgangsloch eine Materialverdickung aufweisen. Diese Materialverdikkung kann bereits vor dem Setzen der Knochenschraube (oder Gewinde-Verformen) vorhanden sein bzw. sich beim Eindrehen einer Knochenschraube durch Materialverdrängung ergeben. Bevorzugt ist die Materialverdickung an der Unterseite des Verbindungsträgers ausgebildet. Zusätzlich oder statt dessen kann sie aber auch an der Oberseite des Verbindungsträgers ausgebildet sein. Eine Materialverdickung an der Unterseite des Verbindungsträgers gewährleistet einen punktförmigen Kontakt zwischen Verbindungsträger und Knochen, so daß in den kontaktfreien Bereichen die Gefäßversorgung der Knochenoberfläche und damit der Heilungsprozeß verbessert wird. Darüber hinaus wird durch die Materialverdickung das Eintauchen des Schraubenkopfes in den Verbindungsträger erleichtert, so daß die Gefahr, daß Schraubenkopfteile überstehen, geringer ist.

Vornehmlich weist die Knochenschraube einen Schaft mit einem Schaftgewinde zum Eindrehen in den Knochen auf. Das Schaftgewinde kann ein selbstschneidendes Gewinde sein, so daß ein Gewindeschneiden mittels eines besonderen Werkzeuges entfallen kann. Die Steigung des vorgeformten Gewindes kann geringfügig kleiner als die des Schaftgewindes der Knochenschraube sein, um neben der Winkelstabilität einen Anpreßdruck des Verbindungsträgers gegen die Knochenoberfläche zu erreichen. Dieses stellt einen zusätzlichen wichtigen Stabilitätsgewinn in der Gesamtverbindung dar.

Zudem kann die Knochenschraube eine Bohrspitze aufweisen, so daß ein Vorbohren eines Loches im Knochen entfallen kann. Für ein leichtes Eindrehen kann der Schraubenkopf einen Werkzeugangriff aufweisen, beispielsweise einen Sechskantinbus. Weiterhin ist die Anordnung konischer Furchen an der Oberfläche des Schraubenkopfes vorteilhaft, da diese eine Aufnahme des umgeformten Materials ermöglichen und so das Einbringen der Knochenschraube in das Durchgangsloch des Verbindungsträger erleichtern.

Gemäß einer weiteren Ausgestaltung hat das Durchgangsloch eine längliche Form und ist der obere Rand des Durchgangsloches von einem maximalen Niveau am einen Ende zu einem minimalen Niveau am anderen Ende geneigt, wobei die Breite des Durchgangsloches vom einen zum anderen Ende hin abnimmt, so daß eine in der Nähe des einen Endes eingeführte Knochenschraube mit dem Schraubenkopf auf dem geneigten Rand aufsitzt und bei Verschraubung unter Verschiebung des Verbindungsträgers mit der Kopfunterseite auf dem geneigten oberen Rand gleitet, bis bei Annäherung an das andere Ende das vorgeformte Gewinde eine Gewindeverbindung bildet. Dabei kann der Rand in der Nähe des anderen Endes den umformbaren Vorsprung unterhalb der darunter angeordneten Sitzfläche bilden. Durch Eindrehen der Knochenschraube wird somit eine Verschiebung des Verbindungsträgers und eine einhergehende zusätzliche Kompression der Fragmentenden erreicht. Bekannt ist, daß durch eine derartige Kompression von zwei Fragmenten die Stabilität ebenfalls erhöht wird. Durch diesen Vorgang werden auch störende Spalten, die von der Knochenheilung überbrückt werden müssen, primär vermieden.

Eine weitere Verbesserung stellt die etwas schräge Anordnung von Durchgangslöchern im Verbindungsträger auf verschiedenen Seiten einer zu überbrückenden Fraktur dar. Diese Schrägstellung der distalen und proximalen Durchgangslöcher zueinander ermöglicht, daß die Knochenschrauben beim Einbringen divergieren, d. h. mit ihren Schraubenköpfen näher zusammen sind als mit ihren "Füßen" (bzw. einer dort angeordneten Bohrspitze). Durch diese divergierende Schraubenlage im Verhältnis zur Achse des Verbindungsträgers und des Knochens kommt es zu einer erhöhten Stabilität und einem besseren Kraftfluß bei Belastung über Verbindungsträger und Knochen.

Bei dem Verbindungsträger kann es sich insbesondere um eine Knochenplatte, um einen Marknagel oder um einen Fixateur handeln.

Vorzugsweise besteht das vorgeformte Gewinde aus einem härteren Material als der von diesem umzuformende Bereich, insbesondere als ein umformbarer Vorsprung. Dafür kann die Knochenschraube oder deren Mantel aus einem härteren Material als der Verbindungsträger oder der umzuformende Bereich desselben sein. Hierdurch wird das Herstellen der Gewindeverbindung weiter erleichtert und dessen Festigkeit weiter verbessert.

Das Fixationssystem nach Anspruch 25 hat einen mindestens ein Durchgangsloch aufweisenden Marknagel zum Überbrücken von Knochenfragementen und mindestens einen Verriegelungsbolzen zum Einschrauben in das Durchgangsloch und in ein Knochenfragment, um dieses mit dem Marknagel zu verbinden. Ferner weist das Durchgangsloch ein Innengewinde zum Einformen eines Gewindes in den Verriegelungsbolzen auf. Der Verriegelungsbolzen hat hingegen einen das Einformen eines Gewindes durch das Innengewinde in verschiedenen Winkeln erleichternden, das Entstehen von Abrieb und Spänen mindernden und das Festsetzen in dem eingeformten Gewinde sicherstellenden Mantelbereich. Dieser ist gewindelos und - zur Förderung der Eindrehbarkeit in verschiedenen Winkeln - konisch und hat eine geringere Härte als der Kernbereich des Verriegelungsbolzens, der die Übertragung hoher Kräfte über den Verriegelungsbolzen gewährleistet. Zur weiteren Förderung der Gewindeausbildung und -sicherung kann der Mantelbereich des Verriegelungsbolzens eine geringere Härte als der Marknagel zumindest im Bereich des Innengewindes haben. Bevorzugt kann überdies der Verriegelungsbolzen beidseits des konischen Mantelbereichs Gewinde mit verschiedenen Außendurchmessern zum Einschrauben in den Knochen tragen. Dabei ist der größere Außendurchmesser auf der Seite des konischen Mantelbereiches mit dem größeren Außendurchmesser angeordnet, um das Einschrauben des Verriegelungsbolzens in Knochen und Marknagel zu ermöglichen bzw. zu begünstigen.

Zwecks weiterer Verringerung des Eindrehmomentes und Minderung des Anfalls von Abrieb und Spänen kann das Durchgangsloch in einem Bereich des Marknagels verringerter Wandstärke ausgebildet sein. Hierdurch wird zudem der Bereich der möglichen Winkelausrichtungen des Verriegelungsbolzens zum Marknagel vergrößert. Zu diesem Zwecke kann das Gewinde im Durchgangsloch überdies nur wenige vollständige Windungen haben, insbesondere ein bis vier vollständig umlaufende Windungen. Ferner kann hierfür an dem gewindetragenden Bereich des Durchgangsloches, der vorzugsweise ballig ausgebildet sein kann, auf mindestens einer Seite des Marknagels ein sphärisch oder konisch nach außen sich erweiternder Bereich des Durchgangsloches angeschlossen sein. Zur Sicherung des Verriegelungsbolzens in seiner Winkelstellung kann dieser überdies im Mantelbereich eine Schulter aufweisen, die als Sitzfläche mit dem ebenfalls eine Sitzfläche bildenden sphärischen oder konischen Bereich des Durchgangsloches zusammenwirkt. Hierdurch, aber auch durch die konische Ausbildung des Mantelbereiches und gegebenenfalls der Knochengewinde wird eine Sicherung des Verriegelungsbolzens erreicht, die eine Sicherung durch Abstützung eines Bolzenkopfes an der Knochenoberfläche mehr oder weniger überflüssig macht. Für das Setzen des Verriegelungsbolzens ist es jedenfalls vorteilhaft, wenn dieser einen Kopf mit einem Werkzeugangriff hat, der grundsätzlich jedoch nicht über den Knochen hinausstehen muß.

Nach einer Weiterbildung der Erfindung weist der Verbindungsträger einen Sensor - z.B. einen Dehnungsmeßstreifen ("DMS")- für die Ermittlung der von dem Verbindungsträger zwischen den Knochenenden übertragenen Kraft und einen Sensor für die telemetrische Übermittlung der Meßergebnisse auf. Sensor und/oder Sender können in einen Hohlraum des Verbindungsträgers integriert sein. Hierdurch wird eine Überwachung der Belastung des Knochens und damit eine Kontrolle des Heilungsprozesses ermöglicht und werden Korrekturen des Verhaltens des Patienten erleichtert.

Vorzugsweise bestehen Knochenschraube, Verriegelungsbolzen und/oder Verbindungsträger aus Titan, wobei für Knochenschraube, Verriegelungsbolzen und Verbindungsträger bzw. Teile dieser Systemkomponenten Titane mit verschiedenen Materialeigenschaften zum Einsatz kommen können.

Zwecks Verbesserung der Zentrierung der Knochenschraube beim Eindrehen in den Knochen kann auf die Sitzfläche des Verbindungsträgers eine Bohrlehre des Fixationssystems mit einer zur Sitzfläche komplementären Kontur gesetzt werden. Durch ein Bohrloch der Bohrlehre kann dann ein Loch im Knochen vorgebohrt werden. Hierdurch wird die zentrale primäre Lage der Sitzfläche der Knochenschraube beim Eindrehen der Schraube in das Durchgangsloch in den Knochen gewährleistet.

Weitere Einzelheiten und Vorteile der Erfindung ergeben sich aus der nachfolgenden Beschreibung der anliegenden Zeichnungen bevorzugter Ausführungsformen. In den Zeichnungen zeigen:
- Fig. 1: eine Knochenschraube in der Vorderansicht;
- Fig. 2a bis i: verschieden ausgeformte Durchgangslöcher in einem Teilschnitt durch einen Verbindungsträger;
- Fig. 3: längliches Durchgangsloch in der Draufsicht auf einen Teil eines Verbindungsträgers;
- Fig. 4: längliches Durchgangsloch in einem teilweisen Längsschnitt durch den Verbindungsträger;
- Fig. 5: dasselbe längliche Durchgangsloch mit eingesetzter Knochenschraube in zwei Verschiebestellungen in einem teilweisen Längsschnitt durch einen Verbindungsträger und einem darunter befindlichen Knochen;
- Fig. 6: einen Marknagel mit Verriegelungsbolzen in einem Röhrenknochen im schematischen Längsschnitt;
- Fig. 7: einen Verriegelungsbolzen mit Schulter im Mantelbereich an einer Sitzfläche im Durchgangsloch des Marknagels in einem Teilschnitt.

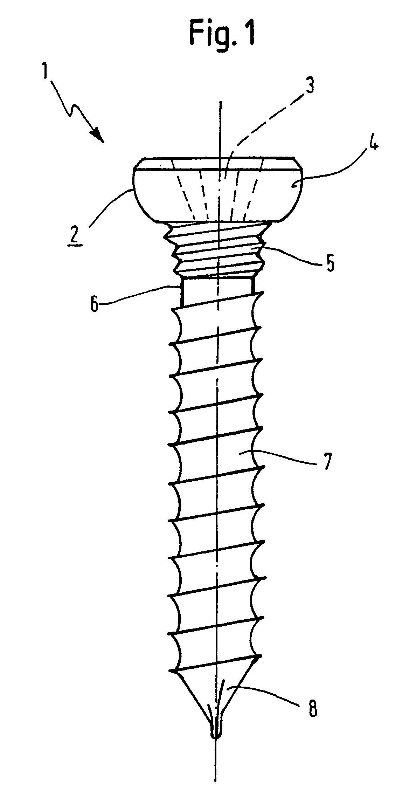

Die Knochenschraube 1 gemäß Fig. 1 hat einen Schraubenkopf 2, der oben einen Sechskantinbus 3 aufweist, unten eine sphärische Sitzfläche 4 hat und darunter ein kurzes vorgeformtes Gewinde 5. Darunter schließt sich ein Schraubenschaft 6 an. Dieser trägt ein Knochengewinde 7 sowie eine Schraubenspitze 8, wobei die Schraubenspitze 8 auch als Bohrspitze ausgeprägt sein kann, so daß sie selbstbohrend für den Knochen ist, und das Knochengewinde selbstgewindeschneidend ausgebildet sein kann.

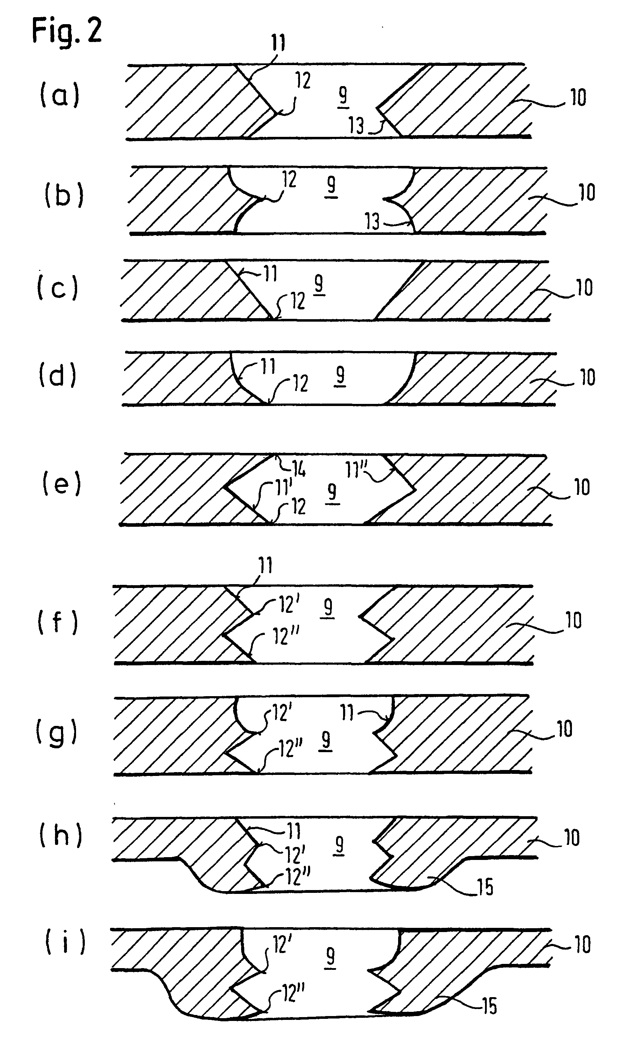

Fig. 2a bis i zeigen unterschiedliche Ausgestaltungen von Durchgangslöchern 9 in Verbindungsträgern 10, die jeweils als Knochenplatten ausgebildet sind.

Gemäß Fig. 2a ist unterhalb einer konischen Sitzfläche 11 etwa mittig im Durchgangsloch 9 ein kreisringförmiger Vorsprung (oder Grat) 12 im Durchgangsloch 9 angeordnet. Darunter hat das Durchgangsloch 9 wiederum eine konische Erweiterung 13.

Gemäß Fig. 2b ist im Durchgangsloch 9 oben eine konische Sitzfläche ausgebildet, die in einen kreisringförmig umlaufenden Vorsprung 12 etwa in der Mitte des Durchgangsloches 9 übergeht. Darunter hat das Durchgangsloch 9 eine sphärische Erweiterung 13.

Fig. 2c und Fig. 2d zeigen Durchgangslöcher 9, an deren Basis ein Vorsprung 12 angeordnet ist. Dabei begrenzt der Vorsprung 12 unten eine konische Sitzfläche 11 (Fig. 2c) bzw. eine sphärische Sitzfläche 11 (Fig. 2d).

Gemäß Fig. 2d ist das Durchgangsloch 9 am unteren Ende von einem Vorsprung 12 und am oberen Ende von einem weiteren Vorsprung 14 begrenzt. Der Vorsprung 12 ist am unteren Ende einer sich nach unten verjüngenden, unteren Sitzfläche 11' und der obere Vorsprung 14 am oberen Ende einer sich nach oben hin verjüngenden, oberen Sitzfläche 11" ausgebildet. Im Querschnitt hat somit das Durchgangsloch 9 im Verbindungsträger 10 eine "Schwalbenschwanz"-Form.

Gemäß Fig. 2f können auch unterhalb einer konusförmigen Sitzfläche 11 zwei Vorsprünge 12', 12" vorhanden sein, von denen einer 12' zugleich das Ende der Sitzfläche darstellt und der weitere Vorsprung 12" in einem axialen Abstand von dem erstgenannten Vorsprung 12' angeordnet ist.

Davon unterscheidet sich das Durchgangsloch 9 gemäß Fig. 2g lediglich dadurch, daß die Sitzfläche 11 oberhalb der beiden Vorsprünge 12', 12" sphärisch ausgebildet ist.

Bei der Ausführung gemäß Fig. 2h hat das Durchgangsloch 9 im Unterschied zu der Ausführung gemäß Fig. 2f eine wulstförmige Materialverdickung 15, die an der Unterseite des Verbindungsträgers 10 angeordnet ist. Auch bei dieser Ausführung befinden sich unterhalb einer sich nach unten verjüngenden, konischen Sitzfläche 11 ein diesen begrenzender Vorsprung 12' und in einem Abstand darunter ein weiterer Vorsprung 12".

Bei der Ausführungsform gemäß Fig. 2i ist im Unterschied zu der Ausführung gemäß Fig. 2g ebenfalls eine wulstige Materialverdickung um das Durchgangsloch 9 angeordnet, die wiederum an der Unterseite des Verbindungsträgers 10 angeordnet ist. Auch hier ist eine sphärische Sitzfläche 11 von einem Vorsprung 12' begrenzt und befindet sich in einem Abstand unter diesem ein weiterer Vorsprung 12".

Eine Knochenschraube 1 ist in verschiedenen Winkelstellungen bezüglich der Achse der Durchgangslöcher 9 der Ausführungen eines Verbindungsträgers 10 gemäß Fig. 2a bis 2i eindrehbar. Dabei formt das vorgeformte Gewinde 5 die Vorsprünge 12 bzw. 12' und 12" um, so daß eine Gewindeverbindung zwischen Knochenschraube 1 und Durchgangsloch 9 gebildet wird, die genau in der Einschraubachse orientiert ist. Beim Umformen füllt das Material der Vorsprünge 12 Hohlräume des vorgeformten Gewindes 3 bzw. neben den Vorsprüngen 12 aus, so daß sich eine Gewindeverbindung im verbreiterten Tragquerschnitt ergibt. Schließlich sitzt die Sitzfläche 4 an der Unterseite des Kugelkopfes 2 auf der Sitzfläche 11 einer der Ausführungen auf und wird von dieser in dem jeweiligen Einschraubwinkel abgestützt. Der Schraubenkopf 2 ragt dann über das Niveau der Oberseite des Verbindungsträgers 10 nicht hinaus. Außerdem wird die Knochenschraube 1 in der erreichten Endstellung durch eine kraft- bzw. reibschlüssige Verbindung mit dem jeweiligen Verbindungsträger 10 im Bereich des Vorsprunges 12 (bzw. der Vorsprünge 12', 12") gesichert.

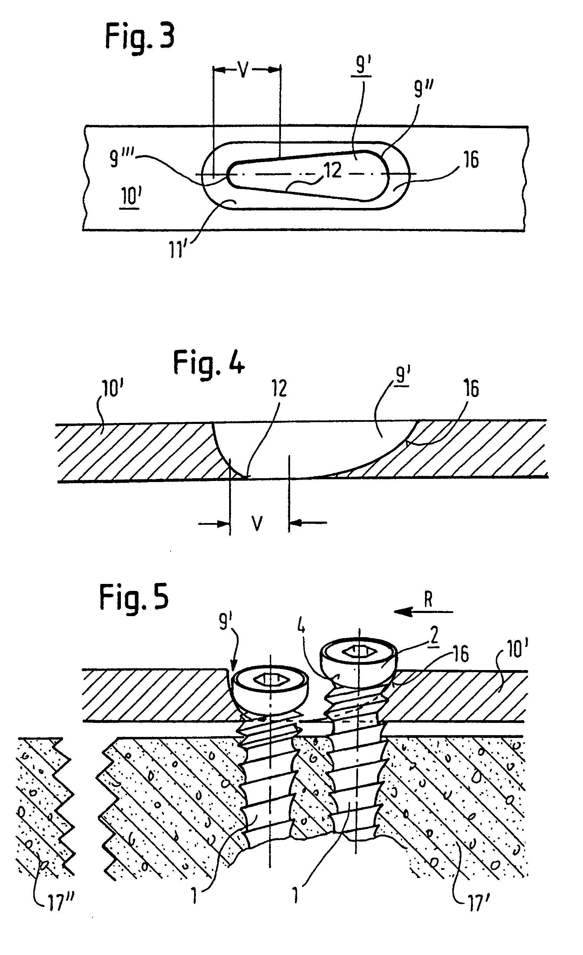

Die Fig. 3 bis 5 zeigen eine längliche Ausgestaltung eines Durchgangsloches 9' einer Knochenplatte 10' mit winkelstabiler Knochenschrauben-Knochenplattenverbindung. Das Durchgangsloch 9' hat eine längliche Ausdehnung in Richtung der Hauptachse der Knochenplatte 10'. Es verjüngt sich von seinem einen Ende 9" zu seinem Ende 9"'. Des weiteren hat es einen oberen Rand 16, der vom einen Ende 9" zum anderen Ende 9''' geneigt ist. Seitlich des oberen Randes 16 ist eine im wesentlichen sphärische Sitzfläche 11' ausgebildet, die um das Ende 9''' voll ausgeprägt ist. Dort geht der Rand 16 in einem Bereich V in einen leicht umformbaren Vorsprung 12 über, der zugleich das Durchgangsloch 9' unten begrenzt.

Gemäß Fig. 5 gleitet eine in das Durchgangsloch 9' eingesetzte Schraube mit der Sitzfläche 4 ihres Schraubenkopfes 2 auf dem Rand 16 bzw. der diese umgebenden Sitzfläche 11' beim Eindrehen in ein Knochenfragment 17' nach unten, wodurch sich eine Verschiebung der Knochenplatte 10' bezüglich des Knochens in Richtung R ergibt und das Knochenfragment 17' gegen ein benachbartes Knochenfragment 17" gedrückt wird. Wenn der Schraubenkopf 2 auf der Sitzfläche 11' aufsitzt und vollständig im Durchgangsloch 9' enthalten ist, hat das vorgeformte Gewinde 5 durch Umformen des Vorsprunges 12 eine winkelstabile Schraubverbindung gebildet. In der dann erreichten Kompressionsstellung werden die Knochenfragmente 17', 17" zusammengedrückt gehalten.

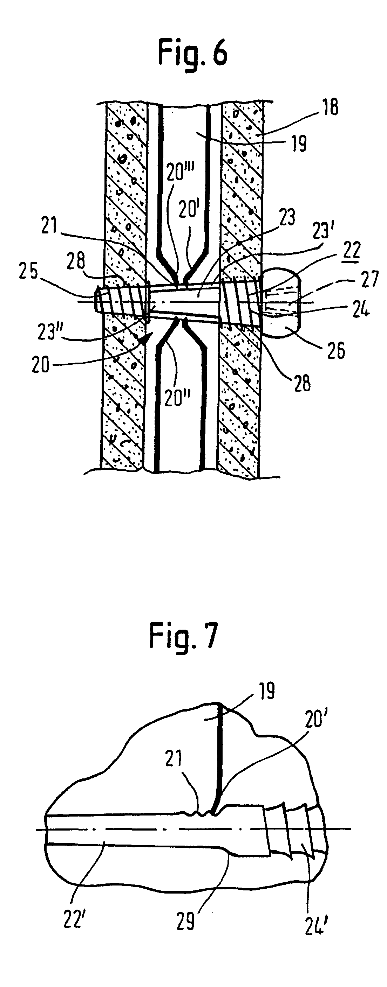

Gemäß Fig. 6 ist in einen Röhrenknochen 18, der an nicht gezeigter Stelle eine Fraktur aufweist, ein Marknagel 19 eingesetzt. Dieser weist auf beiden Seiten der Fraktur mindestens ein Durchgangsloch 20 auf. Das Durchgangsloch 20 hat auf beiden Seiten sphärisch nach außen sich erweiternde Bereiche 20', 20" und in der Mitte einen gewindetragenden Bereich 20"'. Der gewindetragende Bereich 20''' ist zur Mittelachse des Durchgangsloches 20 hin konvex gekrümmt bzw. gerundet. Sein Gewinde 21 hat etwa drei vollständig umlaufende Windungen. Der gesamte Marknagel 19 kann aus einem verhältnismäßig harten Titan bestehen.

In die Durchgangslöcher 20 beidseitig der Fraktur sind Verriegelungsbolzen 22 eingesetzt. Ein Verriegelungsbolzen 22 hat einen insgesamt konischen Mittelabschnitt 23, der einen außen konischen Mantelbereich 23' auf einem außen konischen Kernbereich 23" hat. Beidseits des konischen Mittelabschnittes 23 weist der Verriegelungsbolzen 22 Gewinde 24, 25 zum Einschrauben in den Knochen auf, wobei das Gewinde 24 am Ende des Mittelabschnittes 23 mit dem größeren Außendurchmesser einen größeren Außendurchmesser als das Gewinde 25 auf der anderen Seite hat. Die Außendurchmesser der Gewinde 24, 25 sind jeweils mindestens so groß wie die angrenzenden Außendurchmesser des konischen Mittelabschnittes 23.

Am anderen Ende des Gewindes 24 hat der Verriegelungsbolzen 22 einen Kopf 26, in dem ein Werkzeugangriff in Form eines Inbus 27 ausgebildet ist.

Der Verriegelungsbolzen 22 ist ebenfalls vollständig aus Titan gefertigt. Dabei bestehen der Kernbereich 22", die Knochengewinde 24, 25 und der Kopf 26 aus einem härteren Titanmaterial als der Mantelbereich 23'.

Wenn der Marknagel 19 gesetzt und die Fragmente des Knochens 18 ausgerichtet sind, werden mit Hilfe einer Röntgenapparatur die Lagen der Durchgangslöcher 20 festgestellt und dort Bohrlöcher 28 quer durch den Röhrenknochen 18 in das jeweilige Durchgangsloch 20 eingebracht. Danach werden die Verriegelungsbolzen 22 eingedreht, wobei diese mittels der Gewinde 24, 25 die Gewinde im Knochen 18 selber schneiden können. Ferner formt das Gewinde 21 ein Gewinde in den Mantelbereich 23', wobei die Festigkeit dieser Gewindeverbindung mit zunehmendem Schraubfortschritt aufgrund der konischen Form des Mantelbereiches 23' ansteigt. Eine zusätzliche Sicherung kann durch eine grundsätzlich konische Form der Gewinde 24, 25 erreicht werden und durch eine Auflage des Kopfes 26 des Verriegelungsbolzens 22 an der Außenseite des Knochens 18. Verschiedene Winkelausrichtungen des Verriegelungsbolzens 22, die sich beispielsweise aufgrund von Zielfehlern bei der Anbringung des Bohrloches 28 ergeben können, werden durch die Ausbildung der Durchgangslöcher 20 und Mittelabschnitte 23 ausgeglichen.

Gemäß Fig. 7 kann ein Verriegelungsbolzen 22' unterhalb des Gewindes 24' mit dem größeren Durchmesser eine Schulter 29 aufweisen, die eine Sitzfläche bildet, welche sich gegen den sphärischen Bereich 20' des Durchgangsloches 20 legt, der ebenfalls eine Sitzfläche bildet. Hierdurch wird eine weitere Verbesserung des Sitzes des Verriegelungsbolzens 22' in Knochen 18 und Marknagel 19 erreicht.

## Patentansprüche

1. Fixationssystem für Knochen mit einem Verbindungsträger (10) mit wenigstens einem Durchgangsloch (9), wenigstens einer in ein Durchgangsloch (9) eingesetzten Knochenschraube (1), eine gegenseitige Ausrichtung unter verschiedenen Winkeln ermöglichenden Sitzflächen (4, 11) von Verbindungsträger (10) und Knochenschraube (1) und Mitteln (5, 12) zum Festlegen der Knochenschraube (1) in einem bestimmten Winkel zum Verbindungsträger (10), **dadurch gekennzeichnet, daß** die Mittel (5, 12) zum Festlegen eine durch Eindrehen der Knochenschraube (1) in einem bestimmten Winkel von einem vorgeformten Gewinde (5) unterhalb mindestens einer der Sitzflächen (4) durch Umformung gebildeten Gewindeverbindung unterhalb der Sitzflächen (4, 11) von Knochenschraube (1) und Verbindungsträger (10) aufweisen.

2. Fixationssystem nach Anspruch 1, **dadurch gekennzeichnet, daß** die Gewindeverbindung von einem vorgeformten Gewinde (5) unterhalb der einen Sitzfläche (4) und mindestens einem mindestens teilweise umlaufenden, durch Eindrehen des vorgeformten Gewindes (5) umformbaren Vorsprung (12) unterhalb der anderen Sitzfläche gebildet ist.

3. Fixationssystem nach Anspruch 1 oder 2, **dadurch gekennzeichnet, daß** die Sitzflächen (4, 11) sphärisch oder konisch sind.

4. Fixationssystem nach einem der Ansprüche 1 bis 3, **dadurch gekennzeichnet, daß** die eine Sitzfläche (4) an der Unterseite eines Schraubenkopfes (2) der Knochenschraube (1) ausgebildet ist.

5. Fixationssystem nach einem der Ansprüche 1 bis 4, **dadurch gekennzeichnet, daß** die Gewindeverbindung mit einem Abschnitt des Schraubenkopfes (2) unterhalb der einen Sitzfläche (4) gebildet ist.

6. Fixationssystem nach Anspruch 5, **dadurch gekennzeichnet, daß** die Gewindeverbindung mit einem vorgeformten Gewinde (5) am Schraubenkopf (2) unterhalb der einen Sitzfläche (4) gebildet ist.

7. Fixationssystem nach einem der Ansprüche 1 bis 6, **dadurch gekennzeichnet, daß** die andere Sitzfläche (11) in dem Durchgangsloch (9) des Verbindungsträgers (10) ausgebildet ist.

8. Fixationssystem nach einem der Ansprüche 1 bis 7, **dadurch gekennzeichnet, daß** die Gewindeverbindung mit einem Abschnitt (12) des Durchgangsloches (9) unterhalb der anderen Sitzfläche (11) gebildet ist.

9. Fixationssystem nach Anspruch 8, **dadurch gekennzeichnet, daß** die Gewindeverbindung mit einem umformbaren Vorsprung (12) im Durchgangsloch (9) unterhalb der anderen Sitzfläche (11) gebildet ist.

10. Fixationssystem nach Anspruch 9, **dadurch gekennzeichnet, daß** in einem Abstand von dem umformbaren Vorsprung (12') mindestens ein weiterer, mindestens teilweise umlaufender, durch Eindrehen des vorgeformten Gewindes umformbarer Vorsprung (12") unterhalb der anderen Sitzfläche (11) angeordnet ist.

11. Fixationssystem nach einem der Ansprüche 7 bis 10, **dadurch gekennzeichnet, daß** oberhalb der anderen Sitzfläche (11') im Durchgangsloch (9) mindestens noch ein weiterer, mindestens teilweise umlaufender, durch Eindrehen der Knochenschraube umformbarer Vorsprung (14) angeordnet ist.

12. Fixationssystem nach einem der Ansprüche 1 bis 11, **dadurch gekennzeichnet, daß** der Verbindungsträger (10) um das Durchgangsloch (9) eine Materialverdickung (15) aufweist.

13. Fixationssystem nach Anspruch 12, **dadurch gekennzeichnet, daß** eine Materialverdickung (15) an der Unterseite und/oder an der Oberseite des Verbindungsträgers (10) ausgebildet ist.

14. Fixationssystem nach einem der Ansprüche l bis 13, **dadurch gekennzeichnet, daß** die Knochenschraube (1) einen Schaft (6) mit einem Schaftgewinde (7) aufweist.

15. Fixationssystem nach einem der Ansprüche 1 bis 14, **dadurch gekennzeichnet, daß** das Schaftgewinde (7) ein selbstschneidendes Gewinde ist.

16. Fixationssystem nach Anspruch 14 oder 15, **dadurch gekennzeichnet, daß** die Steigung des vorgeformten Gewindes (5) geringfügig kleiner als die Steigung des Schaftgewindes (7) der Knochenschraube (1) ist.

17. Fixationssystem nach einem der Ansprüche 1 bis 16, **dadurch gekennzeichnet, daß** die Knochenschraube (1) eine Bohrspitze (8) aufweist.

18. Fixationssystem nach einem der Ansprüche 1 bis 17, **dadurch gekennzeichnet, daß** der Schraubenkopf (2) einen Werkzeugangriff (3) aufweist.

19. Fixationssystem nach einem der Ansprüche 1 bis 18, **dadurch gekennzeichnet, daß** der Schraubenkopf (2) an der Unterseite konische Furchen für eine Aufnahme des umgeformten Materials aufweist.

20. Fixationssystem nach einem der Ansprüche 1 bis 19, **dadurch gekennzeichnet, daß** das Durchgangsloch (9') eine längliche Form hat und der obere Rand (16) des Durchgangsloches (9') von einem maximalen Niveau am einen Ende (9") zu einem minimalen Niveau am anderen Ende (9''') geneigt ist, wobei die Breite des Durchgangsloches (9') vom einen zum anderen Ende hin abnimmt, so daß eine in der Nähe des einen Endes (9") eingeführte Knochenschraube (1) mit dem Schraubenkopf (2) auf dem geneigten Rand (16) aufsitzt und bei Verschraubung unter Verschiebung des Verbindungsträgers (10') mit der Kopfunterseite auf dem geneigten Rand (16) gleitet, bis bei Annäherung an das andere Ende (9''') das vorgeformte Gewinde (5) mit dem umformbaren Vorsprung (12') eine Gewindeverbindung bildet.

21. Fixationssystem nach einem der Ansprüche 1 bis 20, **dadurch gekennzeichnet, daß** die Achsen der distalen Durchgangslöcher (9) des Verbindungsträgers (10) so zu den Achsen seiner proximalen Durchgangslöcher (9) geneigt sind, daß die distalen und proximalen Knochenschrauben (1) mit ihren Schraubenköpfen (2) näher zusammen sind als mit ihren Füßen.

22. Fixationssystem nach einem der Ansprüche 1 bis 21, **dadurch gekennzeichnet, daß** der Verbindungsträger (10) eine Knochenplatte, ein Marknagel oder Fixateur ist.

23. Fixationssystem nach einem der Ansprüche 1 bis 22, **dadurch gekennzeichnet, daß** das vorgeformte Gewinde (5) aus einem härteren Material als der umformbare Vorsprung (12) besteht.

24. Fixationssystem nach einem der Ansprüche 1 bis 23, **dadurch gekennzeichnet, daß** die Knochenschraube (1) oder deren Mantel aus einem härteren Material als der Verbindungsträger (10) oder der umzuformende Bereich desselben ist.

25. Fixationssystem für Knochen mit einem mindestens ein Durchgangsloch (20) aufweisenden Marknagel (19) zum Überbrücken von Knochenfragmenten und mindestens einem Verriegelungsbolzen (22) zum Einschrauben in das Durchgangsloch (20) und in ein Knochenfragment, um dieses mit dem Marknagel (19) zu verbinden, **dadurch gekennzeichnet, daß** das Durchgangsloch (20) ein Innengewinde (21) zum Einformen eines Gewindes in den Verriegelungsbolzen (22) hat und der Verriegelungsbolzen (22) einen das Einformen eines Gewindes durch das Innengewinde (21) in verschiedenen Winkeln erleichternden, den Abrieb und Spanfreisetzung vermindernden und das Festsetzen in dem eingeformten Gewinde sicherstellenden, gewindelosen und konischen Mantelbereich (23') mit geringerer Härte als sein die Übertragung hoher Kräfte gewährleistender Kernbereich (23") hat.

26. Fixationssystem nach Anspruch 25, **dadurch gekennzeichnet, daß** der Mantelbereich des Verriegelungsbolzens (22) eine geringere Härte hat als der Marknagel (19) zumindest im Bereich (20''') des Innengewindes (21).

27. Fixationssystem nach Anspruch 25 oder 26, **dadurch gekennzeichnet, daß** der Verriegelungsbolzen (22) beidseits des konischen Mantelbereiches (23') Gewinde (24, 25) trägt, wobei der Außendurchmesser des Gewindes (24) neben dem Ende des konischen Mantelbereiches (23') mit dem größeren Durchmesser größer als der Außendurchmesser des Gewindes (25) neben dem Ende des Mantelbereiches (23') mit dem kleineren Durchmesser ist.

28. Fixationssystem nach einem der Ansprüche 25 bis 27, **dadurch gekennzeichnet, daß** das Durchgangsloch (20) in einem Bereich des Marknagels (19) verringerter Wandstärke ausgebildet ist.

29. Fixationssystem nach einem der Ansprüche 25 bis 28, **dadurch gekennzeichnet, daß** das Innengewinde (21) im Durchgangsloch (20) nur wenige vollständige Windungen hat.

30. Fixationssystem nach Anspruch 29, **dadurch gekennzeichnet, daß** das Innengewinde (21) im Durchgangsloch (20) ein bis vier vollständig umlaufende Windungen hat.

31. Fixationssystem nach einem der Ansprüche 25 bis 30, **dadurch gekennzeichnet, daß** an den gewindetragenden Bereich (20') des Durchgangsloches (20) auf mindestens einer Seite des Marknagels (19) ein sphärisch oder konisch nach außen sich erweiternder Bereich (20', 20") des Durchgangsloches (20) anschließt.

32. Fixationssystem nach Anspruch 31, **dadurch gekennzeichnet, daß** der Verriegelungsbolzen (22') eine Schulter (29) aufweist, die eine Sitzfläche für Anlage an dem eine Sitzfläche bildenden sphärischen oder konischen Bereich (20') des Durchgangsloches (20) ist.

33. Fixationssystem nach einem der Ansprüche 25 bis 32, **dadurch gekennzeichnet, daß** der Verriegelungsbolzen (22) einen Kopf (26) mit einem Werkzeugangriff (27) hat.

34. Fixationssystem nach einem der Ansprüche 1 bis 33, **dadurch gekennzeichnet, daß** der Verbindungsträger (10) einen Sensor für die Ermittlung der von dem Verbindungsträger zwischen den Knochenenden übertragenen Kraft und einen Sender für die telemetrische Übermittlung der Meßergebnisse aufweist.

35. Fixationssystem nach Anspruch 34, **dadurch gekennzeichnet, daß** Sensor und/oder Sender in einen Hohlraum des Verbindungsträgers (10) integriert sind.

36. Fixationssystem nach einem der Ansprüche 1 bis 35, **dadurch gekennzeichnet, daß** die Knochenschraube (1) oder der Verriegelungsbolzen und/oder der Verbindungsträger (10) aus Titan besteht.

## Claims

1. A fixation system for bones with a joining support (10) having at least one through hole (9), at least one bone screw (1) inserted into a through hole (9), seating surfaces (4, 11) of the joining support (10) and the bone screw (1) which enable a reciprocal orientation under different angles, and means (5, 12) for locating the bone screw (1) at a certain angle from the joining support (10), **characterized in that** the means (5, 12) for locating have a threaded joint formed by a deformation of a preformed thread (5) by turning in the bone screw (1) at a certain angle below at least one of the seating surfaces (4) of the bone screw (1) and the joining support (10).

2. The fixation system according to claim 1, **characterized in that** the threaded joint is defined by a preformed thread (5) below the one seating surface (4) and at least one at least partially circumferential protrusion (12) deformable by turning in the preformed thread (5) below the other seating surface.

3. The fixation system according to claim 1 or 2, **characterized in that** the seating surfaces (4, 11) are spherical or conical.

4. The fixation system according to any one of claims 1 to 3, **characterized in that** the one seating surface (4) is formed at the underside of a screw head (2) of the bone screw (1).

5. The fixation system according to any one of claims 1 to 4, **characterized in that** the threaded joint is formed with a portion of the screw head (2) below the one seating surface (4).

6. The fixation system according to claim 5, **characterized in that** the threaded joint is formed with a preformed thread (5) on the screw head (2) below the one seating surface (4).

7. The fixation system according to any one of claims 1 to 6, **characterized in that** the other seating surface (11) is formed in the through hole (9) of the joining support (10).

8. The fixation system according to any one of claims 1 to 7, **characterized in that** the threaded joint is formed with a portion (12) of the through hole (9) below the other seating surface (11).

9. The fixation system according to claim 8, **characterized in that** the threaded joint is formed with a deformable protrusion (12) in the through hole (9) below the other seating surface (11).

10. The fixation system according to claim 9, **characterized in that** at least one further at least partially circumferential protrusion (12") deformable by turning in the preformed thread is disposed at a distance from the deformable protrusion (12') below the other seating surface (11).

11. The fixation system according to any one of claims 7 to 10, **characterized in that** at least one further at least partially circumferential protrusion (14) deformable by turning in the bone screw is disposed in the through hole above the other seating surface (11').

12. The fixation system according to any one of claims 1 to 11, **characterized in that** the joining support (10) has a thickened material region (15) around the through hole (9).

13. The fixation system according to claim 12, **characterized in that** a thickened material region (15) is formed at the underside and/or the upper side of the joining support (10).

14. The fixation system according to any one of claims 1 to 13, **characterized in that** the bone screw (1) has a shank (6) including a shank thread (7).

15. The fixation system according to any one of claims 1 to 14, **characterized in that** the shank thread (7) is a self-cutting thread.

16. The fixation system according to claim 14 or 15, **characterized in that** the pitch of the preformed thread is slightly smaller than the pitch of the shank thread (7) of the bone screw (1).

17. The fixation system according to any one of claims 1 to 16, **characterized in that** the bone screw (1) has a drilling tip (8).

18. The fixation system according to any one of claims 1 to 17, **characterized in that** the screw head (2) has a tool receptacle (3).

19. The fixation system according to any one of claims 1 to 18, **characterized in that** the screw head (2) has conical ridges at the underside to receive the deformed material.

20. The fixation system according to any one of claims 1 to 19, **characterized in that** the through hole (9') is of an oblong shape and the upper border (16) of the through hole (9') is inclined from a maximum level at one end (9") towards a minimum level at the other end (9''') wherein the width of the through hole (9') diminishes from one end to the other so that a bone screw (1) inserted in the vicinity of one end (9") seats the screw head (2) on the inclined border (16) and, if screwed together, causes its head underside to slide on the inclined border (16) while displacing the joining support (10') until the preformed thread (5), while approaching the other end (9'''), forms a threaded joint with the deformable protrusion (12').

21. The fixation system according to any one of claims 1 to 20, **characterized in that** the axes of the distal through holes (9) of the joining support (10) are inclined towards the axes of its proximal through holes (9) in such a way that the distal and proximal bone screws (1) are closer to each other at their screw heads (2) than at their feet.

22. The fixation system according to any one of claims 1 to 21, **characterized in that** the joining support (10) is a bone plate, marrow nail or fixateur.

23. The fixation system according to any one of claims 1 to 22, **characterized in that** the preformed thread (5) is made of a material harder than that of the deformable protrusion (12).

24. The fixation system according to any one of claims 1 to 23, **characterized in that** the bone screw (1) or its shell is made of a material harder than that of the joining support (10) or the deformable portion thereof.

25. A fixation system for bones including a marrow nail (19) having at least one through hole (20) to span bone fragments and at least one locking pin (22) to be screwed into the through hole (20) and into a bone fragment to join it to the marrow nail (19), **characterized in that** the through hole (20) has a female thread (21) for forming a thread into the locking pin (22) and the locking pin (22) has a non-threaded and conical shell region (23') of a hardness smaller than that of its core region (23") ensuring the transmission of large forces, which makes it easier to form in a thread by the female thread (21) at different angles, to reduce abrasion and chip release, and to ensure a fixation in the formed-in thread.

26. The fixation system according to claim 25, **characterized in that** the shell region of the locking pin (22) is of a hardness smaller than that of the marrow nail at least in the area (20''') of the female thread (21).

27. The fixation system according to claim 25 or 26, **characterized in that** the locking pin (22) carries threads (24, 25) on either side of the conical shell region (23') wherein the outer diameter of the thread (24) next to the end of the conical shell region (23') having the larger diameter is larger than the outer diameter of the thread (25) next to the end of the shell region (23') having the smaller diameter.

28. The fixation system according to any one of claims 25 to 27, **characterized in that** the through hole (20) is formed in a region of reduced wall thickness of the marrow nail (19).

29. The fixation system according to any one of claims 25 to 28, **characterized in that** the female thread (21) in the through hole (20) only has a few complete convolutions.

30. The fixation system according to claim 29, **characterized in that** the female thread (21) in the through hole (20) has one to four completely circumferential convolutions.

31. The fixation system according to any one of claims 25 to 30, **characterized in that** the thread-carrying region (20') of the through hole (20) is joined by a spherically or conically outwardly extending region (20', 20") of the through hole (20) on at least one side of the marrow nail (19).

32. The fixation system according to claim 31, **characterized in that** the locking pin (22') has a shoulder (29) which is a seating surface for abutting the spherical or conical region (20') of the through hole (20) that forms a seating surface.

33. The fixation system according to any one of claims 25 to 32, **characterized in that** the locking pin (22) has a head (26) with a tool receptacle (27).

34. The fixation system according to any one of claims 1 to 33, **characterized in that** the joining support (10) has a sensor for determining the force transferred by the joining sensor between the bone ends and a transmitter for telemetrically transmitting the results measured.

35. The fixation system according to claim 34, **characterized in that** the sensor and/or transmitter are integrated in a cavity of the joining support (10).

36. The fixation system according to any one of claims 1 to 35, **characterized in that** the bone screw (1) or the locking pin and/or the joining support (10) are made of titanium.

## Revendications

1. Système de fixation pour des os, avec un support de liaison (10) ayant au moins un trou de passage (9), au moins une vis à os (1) insérée dans un trou de passage (9), des faces d'assise (4, 11) permettant une orientation mutuelle, sous différents angles, des supports de liaison (10) et de la vis à os (1), et des moyens (5, 12) pour fixer la vis à os (1) sous un angle déterminé par rapport au support de liaison (10), **caractérisé en ce que** les moyens (5, 12) prévus pour assurer la fixation présentent une liaison filetée, formée par reformage obtenu par insertion avec rotation d'un angle déterminé de la vis à os (1), par un filetage (5) préformé situé au-dessous d'au moins l'une des faces d'assise (4), liaison filetée formée au-dessous des faces d'assise (4, 11) de la vis à os (1) et du support de liaison (10).

2. Système de fixation selon la revendication 1, **caractérisé en ce que** la liaison filetée est formée par un filetage (5) préformé, au-dessous de la première des faces d'assise (4), et par au moins une saillie (12), faisant au moins partiellement le pourtour, reformable par insertion accompagnée de rotation du filetage (5) préformé, au-dessous de l'autre face d'assise.

3. Système de fixation selon la revendication 1 ou 2, **caractérisé en ce que** les faces d'assise (4, 11) sont sphériques ou coniques.

4. Système de fixation selon l'une des revendications 1 à 3, **caractérisé en ce que** la première des faces d'assise (4) est réalisée sur la face inférieure d'une tête de vis (2) de la vis à os (1).

5. Système de fixation selon l'une des revendications 1 à 4, **caractérisé en ce que** la liaison filetée est formée avec un tronçon de la tête de vis (2) situé au-dessous de la première des faces d'assise (4).

6. Système de fixation selon la revendication 5, **caractérisé en ce que** la liaison filetée est formée par un filetage (5) préformé sur la tête de vis (2), au-dessous de la première des faces d'assise (4).

7. Système de fixation selon l'une des revendications 1 à 6, **caractérisé en ce que** l'autre face d'assise (11) est formée dans le trou de passage (9) du support de liaison (10).

8. Système de fixation selon l'une des revendications 1 à 7, **caractérisé en ce que** la liaison filetée est formée par un tronçon (12) du trou de passage (9), au-dessous de l'autre face d'assise (11).

9. Système de fixation selon la revendication 8, **caractérisé en ce que** la liaison filetée est formée par une saillie (12) reformable, dans le trou de passage (9), au-dessous de l'autre face d'assise (11).

10. Système de fixation selon la revendication 9, **caractérisé en ce que**, à une certaine distance par rapport à la saillie (12') reformable, au moins une autre saillie (12"), reformable par une insertion accompagnée de rotation du filetage préformé, faisant au moins partiellement le pourtour, est disposée au-dessous de l'autre face d'assise (11).

11. Système de fixation selon l'une des revendications 7 à 10, **caractérisé en ce qu'**au-dessus de l'autre face d'assise (11') est disposé dans le trou de passage (9) encore au moins une autre saillie (14), reformable par insertion accompagné de rotation de la vis à os, faisant au moins partiellement le pourtour.

12. Système de fixation selon l'une des revendications 1 à 11, **caractérisé en ce que** le support de liaison (10) présente un épaississement de matériau (15) autour du trou de passage (9).

13. Système de fixation selon la revendication 12, **caractérisé en ce qu'**un épaississement de matériau (15) est réalisé sur la face inférieure et/ou sur la face supérieure du support de liaison (10).

14. Système de fixation selon l'une des revendications 1 à 13, **caractérisé en ce que** la vis à os (1) présente une tige (6) munie d'un filetage de tige (7).

15. Système de fixation selon l'une des revendications 1 à 14, **caractérisé en ce que** le filetage de tige (7) est un filetage auto-taraudant.

16. Système de fixation selon la revendication 14 ou 15, **caractérisé en ce que** le pas du filetage (5) préformé est légèrement inférieur au pas du filetage de tige (7) de la vis à os (1).

17. Système de fixation selon l'une des revendications 1 à 16, **caractérisé en ce que** la vis à os (1) présente une pointe de perçage (8).

18. Système de fixation selon l'une des revendications 1 à 17, **caractérisé en ce que** la tête de vis (2) présente une emprise pour un outil (3).

19. Système de fixation selon l'une des revendications 1 à 18, **caractérisé en ce que** la tête de vis (2) présente en face inférieure des sillons coniques pour recevoir le matériau reformé.

20. Système de fixation selon l'une des revendications 1 à 19, **caractérisé en ce que** le trou de passage (9') a une forme allongée et le bord supérieure (16) du trou de passage (9') est incliné depuis un niveau maximal à une extrémité (9") jusqu'à un niveau minimal à l'autre extrémité (9'''), la largeur du trou de passage (9') allant en diminuant d'une extrémité à l'autre, de manière qu'une vis à os (1) insérée à proximité d'une extrémité (9") repose par la tête de vis (2) sur le bord (16) incliné et, lors du vissage accompagné de déplacement du support de liaison (10'), glisse avec la face inférieure de tête sur le bord (16) incliné, jusqu'à ce que, à l'approché de l'autre extrémité (9'''), le filetage (5) préformé forme avec la saillie (12') reformable une liaison filetée.

21. Système de fixation selon l'une des revendications 1 à 20, **caractérisé en ce que** les axes des trous de passage (9) distaux du support de liaison (10) sont inclinés par rapport aux axes de ses trous de passage (9) proximaux, de manière que les vis à os (1) distales et proximales soient conjointement, par leur tête de vis (2), plus proches que par leur pied.

22. Système de fixation selon l'une des revendications 1 à 21, **caractérisé en ce que** le support de liaison (10) est une plaque à os, un clou d'osthéosynthèse intramédullaire ou bien un fixateur.

23. Système de fixation selon l'une des revendications 1 à 22, **caractérisé en ce que** le filetage (5) préformé est constitué d'un matériau plus dur que celui de la saillie (12) reformable.

24. Système de fixation selon l'une des revendications 1 à 23, **caractérisé en ce que** la vis à os (1) ou bien son enveloppe est formée d'un matériau plus dur que le support de liaison (10) ou bien la zone à reformer de celui-ci.

25. Système de fixation pour os avec au moins un clou d'osthéosynthèse intramédullaire (19) présentant un trou de passage (20), pour assurer le pontage de fragments d'os, et d'au moins un boulon de verrouillage (22), à visser dans le trou de passage (20) et dans un fragment d'os, afin de relier celui-ci au clou d'osthéosynthèse intramédullaire (19), **caractérisé en ce que** le trou de passage (20) comporte un filetage intérieur (21) pour former par pénétration un filetage dans le boulon de verrouillage (22) et le boulon de verrouillage (22) comporte une zone d'enveloppe (23') sans filetage et conique, assurant la diminution de l'abrasion et le dégagement pour le passage des copeaux, ainsi que le blocage dans le filetage ayant été creusé, en facilitant le creusement d'un filetage dans le filetage intérieur, sous différents angles, la zone d'enveloppe (23') ayant une dureté inférieure à sa zone de noyau (23") assurant la transmission d'efforts élevés.

26. Système de fixation selon la revendication 25, **caractérisé en ce que** la zone d'enveloppe du boulon de verrouillage (22) a une dureté inférieure au clou d'osthéosynthèse intramédullaire (19), au moins dans la zone (20''') du filetage intérieur (21).

27. Système de fixation selon la revendication 25 ou 26, **caractérisé en ce que**, des deux côtés de la zone d'enveloppe (23') conique, le boulon de verrouillage (22) porte un filetage (24, 25), le diamètre extérieur du filetage (24), à côté de l'extrémité de la zone d'enveloppe (23') conique, est d'un plus grand diamètre, supérieur au diamètre extérieur du filetage (25) à côté de l'extrémité de la zone d'enveloppe (23') ayant le plus petit diamètre.

28. Système de fixation selon l'une des revendications 25 à 27, **caractérisé en ce que** le trou de passage (20) est réalisé dans une zone où le clou d'osthéosynthèse intramédullaire (19) est d'une épaisseur de paroi diminuée.

29. Système de fixation selon l'une des revendications 25 à 28, **caractérisé en ce que** le filetage intérieur (21) ne présente dans le trou de passage (20) que quelques filets complets.

30. Système de fixation selon la revendication 29, **caractérisé en ce que** le filetage intérieur (21) réalisé dans le trou de passage (20) a un nombre de spires, faisant complètement la circonférence, allant de 1 à 4.

31. Système de fixation selon l'une des revendications 25 à 30, **caractérisé en ce que**, à la zone (20') portant le filetage du trou de passage (20), se raccorde, sur au moins un côté du clou d'osthéosynthèse intramédullaire (19), à une zone (20', 20"), allant en s'élargissant de façon sphérique ou conique vers l'extérieur, du trou de passage (20).

32. Système de fixation selon la revendication 31, **caractérisé en ce que** le boulon de verrouillage (22') présente un épaulement (29) qui est une face d'assise pour la mise en appui sur une zone (20') sphérique ou conique, formant une face d'assise, du trou de passage (20).

33. Système de fixation selon l'une des revendications 25 à 32, **caractérisé en ce que** le boulon de verrouillage (22) a une tête (26) portant une emprise pour un outil (27).

34. Système de fixation selon l'une des revendications 1 à 33, **caractérisé en ce que** le support de liaison (10) présente un capteur, de détermination de la force transmise par le support de liaison entre les extrémités d'os, et un émetteur, permettant la transmission télémétrique des résultats de mesure.

35. Système de fixation selon la revendication 34, **caractérisé en ce que** le capteur et/ou l'émetteur sont intégrés dans un espace creux du support de liaison (10).

36. Système de fixation selon l'une des revendications 1 à 35, **caractérisé en ce que** la vis à os (1) ou le boulon de verrouillage et/ou le support de liaison (10) sont en titane.
